Europäisches Patentamt

European Patent Office

Office européen des brevets

(11) Numéro de publication: **0 280 592**
**A2**

(12) **DEMANDE DE BREVET EUROPEEN**

(21) Numéro de dépôt: **88400154.6**

(22) Date de dépôt: **25.01.88**

(51) Int. Cl.⁴: **B 22 F 7/00**
A 61 F 2/30, A 61 L 27/00

(30) Priorité: **28.01.87 FR 8700991**

(43) Date de publication de la demande:
**31.08.88 Bulletin 88/35**

(84) Etats contractants désignés:
**CH DE ES GB IT LI SE**

(71) Demandeur: **COMMISSARIAT A L'ENERGIE ATOMIQUE**
**31/33, rue de la Fédération**
**F-75015 Paris (FR)**

(72) Inventeur: **Devillard, Jacques**
**474, Chemin du Rosat**
**F-38330 Saint Ismier (FR)**

(74) Mandataire: **Mongrédien, André et al**
**c/o BREVATOME 25, rue de Ponthieu**
**F-75008 Paris (FR)**

(54) Procédé de fixation d'une couche poreuse sur un substrat et application de ce procédé à la réalisation d'une prothèse.

(57) Procédé d'accrochage d'une couche poreuse sur un substrat.

Un substrat (32, 33) destiné à constituer l'âme d'une prothèse de hanche est placé dans une cavité (28) d'un moule (22). Des sphérules (10) sont introduites dans l'espace compris entre le substrat (32, 33) et les parois de la cavité (28). Le moule (22) est placé dans un four (34), que l'on porte à une température à laquelle le matériau constitutif du substrat est superplastique. On applique ensuite au moule (22) une pression de l'ordre de 10 à 30 MPa, ce qui permet d'incruster les sphérules (10) sur le substrat à une température relativement basse.

Application à la réalisation de prothèses osseuses.

FIG. 5

## Description

## PROCEDE DE FIXATION D'UNE COUCHE POREUSE SUR UN SUBSTRAT ET APPLICATION DE CE PROCEDE A LA REALISATION D'UNE PROTHESE

La présente invention se rapporte au domaine de la fixation d'une couche poreuse sur un support rigide dense et trouve une application particulièrement intéressante dans la réalisation de prothèses, notamment des prothèses de hanche.

La plupart des prothèses de hanche se composent d'une tige intramédullaire scellée au fémur par un ciment acrylique. Pendant sa prise, ce dernier met en oeuvre une polymérisation exothermique. Le dégagement de chaleur est important et la température peut atteindre localement des valeurs de l'ordre de 60°C. Il en résulte une première nécrose non négligeable des tissus environnants. De plus, un autre inconvénient de ce type de prothèse vient de ce que, compte tenu du coefficient de dilatation thermique de ce ciment, qui est dix fois plus grand que celui du tissu osseux voisin, il apparaît au refroidissement un jeu entre l'os et le ciment qui peut atteindre à certains endroits plusieurs micromètres. De plus, la couche de ciment se fritte sur la tie métallique de la prothèse et risque de se fissurer. Les débris qui en résultent induisent également à plus ou moins long terme la nécrose du tissu osseux adjacent, nécrose qui conduit elle-même finalement au descellement de la tige.

Pour pallier ces inconvénients, on a proposé plusieurs solutions qui évitent l'emploi du ciment. Ces méthodes consistent généralement à créer sur la queue fémorale, par usinage ou par moulage, des stries ou des rugosités irrégulières qui augmentennt la surface de contact avec le canal médullaire du fémur. Cette solution permet d'éviter l'emploi du ciment, le tissu osseux venant en contact direct avec la prothèse par réhabitation du relief ainsi créé. Cependant, cette réhabitation est incertaine car elle n'a généralement pas lieu dans des conditions optimales et elle reste difficile à maîtriser.

D'autres solutions plus fiables ont été proposées qui utilisent la métallurgie des poudres pour réaliser la zone poreuse.

Le document WO-83/00282 décrit un procédé dans lequel la zone poreuse est obtenue en recouvrant la surface de la prothèse d'un mélange de deux poudres différentes, par exemple de titane et de fer. Après densification partielle, l'une des deux poudres est éliminée, par exemple par dissolution, ce qui laisse des vides importants entre les grains de l'autre poudre.

D'autre part, le document EP-A-0 075 378 décrit un procédé dans lequel la zone poreuse est obtenue directement par frittage avec des poudres de différentes granulométries. On place une première poudre en contact avec la surface de la prothèse correspondant à la queue fémorale et une deuxième poudre en contact avec la partie de la prothèse correspondant à la tête de celle-ci. Par un choix judicieux des poudres et par le contrôle des paramètres de densification, on obtient simultanément une zone poreuse du côté de la queue fémorale et une zone dense du côté de la tête.

Si ces deux dernières solutions sont plus fiables que les précédentes, elles présentent encore certains inconvénients. Ceux-ci sont essentiellement dus au fait que, dans les deux cas, la zone poreuse est liée métallurgiquement au support par diffusion. Or, deux phénomènes contribuent à la dégradation des propriétés mécaniques d'une prothèse de hanche réhabitable par le tissu osseux lorsque cette prothèse est composée d'un support métallique dense (qui peut être constituée de titane, d'un alliage de titane ou d'un alliage à base de cobalt ou de fer) et d'un revêtement poreux adhérant à la surface de ce support. Les températures nécessaires pour réaliser la liaison entre le support et le revêtement, notamment dans le cas des alliages de titane qui sont les plus utilisés, sont élevées (de l'ordre de 1150 à 1450°C). De telles températures conduisent à un grossissement important du grain $\beta$ et à la formation d'un joint de grain $\alpha$, ce qui a pour effet de dégrader les caractéristiques mécaniques du support. Cette température de frittage peut être abaissée à l'aide d'adjuvants de frittage (par exemple le cuivre ou l'argent), ce qui entraîne un déplacement local de la température de transition entre la structure $\alpha/\beta$ et la structure $\beta$. Ceci contribue à la création d'une couche de liaison à structure $\beta$. Ce changement de structure augmente la sensibilité à la propagation des fissures dans les alliages de titane qui sont, d'autre part, très sensibles à l'effet d'entaille, c'est-à-dire à l'initiation d'une fissure.

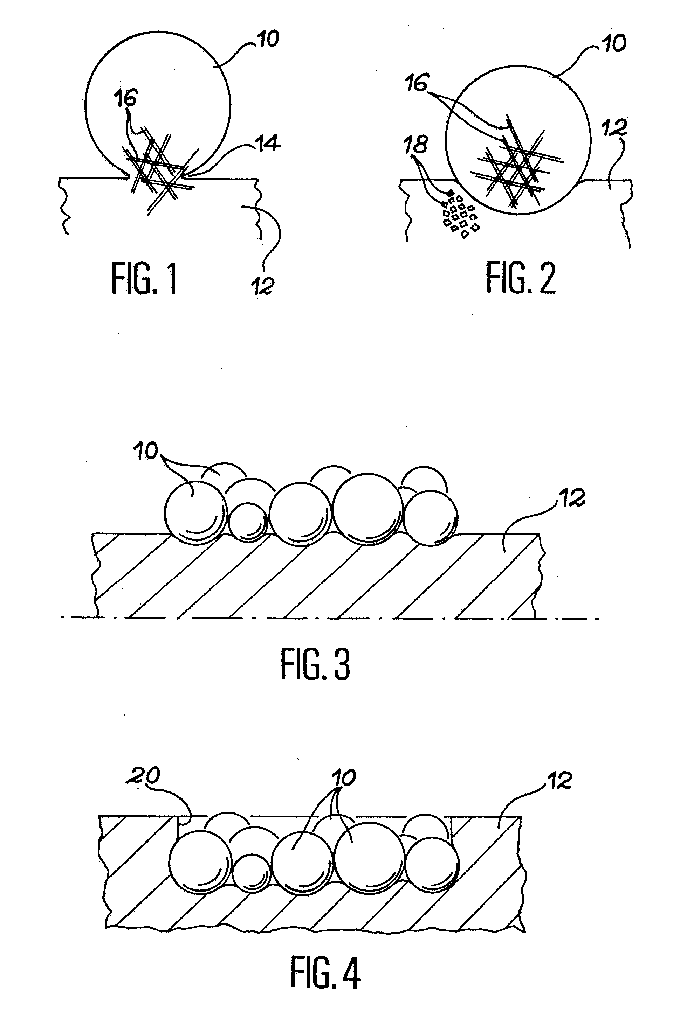

Or, une liaison métallurgique d'une sphérule sur un support en alliage de titane est équivalente à une entaille usinée sur un support lisse. Ceci apparaît sur la figure 1 ci-jointe où l'on voit une sphérule 10 liée métallurgiquement à un substrat 12. On voit que la sphérule 10 se rattache au substrat 12 par un col 14 de très faible rayon. Comme il y a eu diffusion entre la sphérule et le substrat, tout se passe comme s'il s'agissait d'un seul objet dans lequel on aurait ménagé une entaille dont la forme serait celle du col 14.

Le coefficient d'intensité de contrainte équivalent $K_t$ sera d'autant plus élevé que le rayon de raccordement ou le col de frittage entre la sphérule et le support sera de faible dimension.

On observe cette géométrie dans le procédés mettant en oeuvre le frittage naturel (sans contraintes externes) avec ou sans adjuvant de frittage. Dans le cas où l'on utilise un tel adjuvant, la température d'apparition du premier liquide supérieure à la température de transformation de la structure $\alpha$ en structure $\beta$ contribue à la formation de joints de grains $\alpha$ au refroidissement dans le support forgé et à une mise en solution partielle dans les sphérules constituant la zone poreuse. La formation de plaquettes $\alpha$ traversant la zone de diffusion (référence 16 sur la figure 1) contribue à la fragilité de la liaison sous contraintes. Dans le cas d'une couche poreuse liée à un support en alliage de titane, on se trouve confronté au problème de la présence d'entailles dans une structure particulièrement défavorable à la résistance du support. La diminution des caractéristiques mécaniques de ces éléments composites est considérable avec une limite d'endurance pouvant être seulement égale à 20% de celle qu'on mesure sur de tels alliages lorsque l'expérience est réalisée avec des éprouvettes lisses.

La présente invention a pour but de remédier à ces inconvénientes en proposant un procédé de fixation d'une couche poreuse sur un substrat utilisable notamment pour réaliser des prothèses qui permet un accrochage aisé et un bon maintien des sphérules sur le support.

Selon la principale caractéristique du procédé objet de l'invention, le substrat étant en un premier matériau et la couche poreuse en un deuxième matériau, ce procédé comprend les étapes suivantes consistant à :

(a) - amener le substrat dans des conditions dans lesquelles le premier matériau est superplastique, et

(b) - fritter la couche poreuse sur le substrat afin que celle-ci s'incruste au moins partiellement sur la surface du substrat.

De préférence, le premier et deuxième matériaux appartiennent au groupe constitué par : le titane, le cobalt, le chrome, le tantale et leurs alliages.

Selon un autre aspect de l'invention, le couche poreuse est constituée de sphérules dont le diamètre est compris entre 100 et 1000 micromètres, de préférence entre 500 et 600 micromètres.

Lorsque le premier matériau est du titane, l'étape (b) est effectuée à une température comprise entre 700 et 950°C et de préférence à 825°C. Si on utilise d'autres métaux ou alliages, l'homme du métier pourra choisir d'autres températures, l'essentiel étant que le premier matériau soit superplastique à la température utilisée.

Quant à la pression sous laquelle est effectuée l'étape (b), elle est comprise entre 10 et 30 MPa et de préférence égale à 20 MPa.

Avantageusement, ce procédé peut comporter une étape supplémentaire (c), effectuée avant l'étape (b), consistant à créer une barrière antidiffusion à la surface du substrat. Cette barrière antidiffusion peut être obtenue pr création d'une couche d'oxyde à la surface du substrat.

Le procédé peut encore comporter une étape supplémentaire (d), effectuée avant l'étape (b), consistant à fritter la couche poreuse hors substrat à haute température, la fixation sur le substrat intervenant au cours d'un deuxième traitement thermique constitué par l'étape (b) lorsque le premier matériau est dans son domaine de superplasticité. Lorsque le deuxième matériau est du titane, cette étape (d) est effectuée à une température comprise entre 1000 et 1450°C et de préférence à 1250°C. S'il s'agit d'un autre métal ou alliage, on effectuera l'étape (d) à une température à laquelle la couche poreuse peut se fritter hors substrat.

Enfin, ce procédé trouve une application particulièrement intéressante dans le réalisation de prothèses et notamment dans la réalisation des prothèses de hanche.

On rappellera également quelques notions connues de métallurgie concernant ce que l'on désigne couramment par état superplastique.

L'état superplastique, pour un métal donné, est parfaitement reproductible et contrôlé. Il dépend des traitements subis par le matériau lors de son élaboration. Ainsi, les poudres de titane ou d'un de ses alliages présentent une structure aciculaire ne possèdent pas de domaine de superplasticité. Au contraire, une éprouvette de titane ou de l'un de ses alliages, obtenue par coulée puis corroyée ou laminée possède une structure à grains fins équiaxes qui lui confère la propriété de superplasticité dans un domaine de température. Cela est connu de l'homme de métier. Ainsi, les tôles de titane citées dans le brevet EP-A 191 182, sont des matériaux superplastiques.

L'existence de cette propriété n'est pas seulement due à la nature du matériau mais aussi aux traitements métallurgiques qu'il subit.

Dans la présente invention, le support doit être un matériau possédant cette propriété. Le matériau des sphérules formant la couche poreuse ne possède pas de domaine de superplasticité. Ces sphérules sont un produit brut d'atomisation sous vide (procédé PSV).

L'invention apparaîtra mieux à la lecture de la description qui va suivre, donnée à titre d'exemple purement illustratif et nullement limitatif, en référence aux dessins annexés, dans lesquels :

- la figure 1 est une vue schématique à échelle agrandie montrant une sphérule fixée sur un substrat dans les méthodes d'accrochage de couches poreuses de l'art antérieur,

- la figure 2 est une vue semblable à la figure 1 montrant ce qu'on obtient avec le procédé objet de l'invention,

- la figure 3 est une vue schématique en coupe à échelle agrandie montrant une première façon de disposer la couche poreuse constituée de sphérules sur un substrat,

- la figure 4 est une vue semblable à la figure 3 illustrant une autre manière de placer la couche poreuse à la surface du substrat,

- la figure 5 est une vue schématique en coupe montrant un moule placé dans un four pour la mise en oevre du procédé objet de l'invention, et

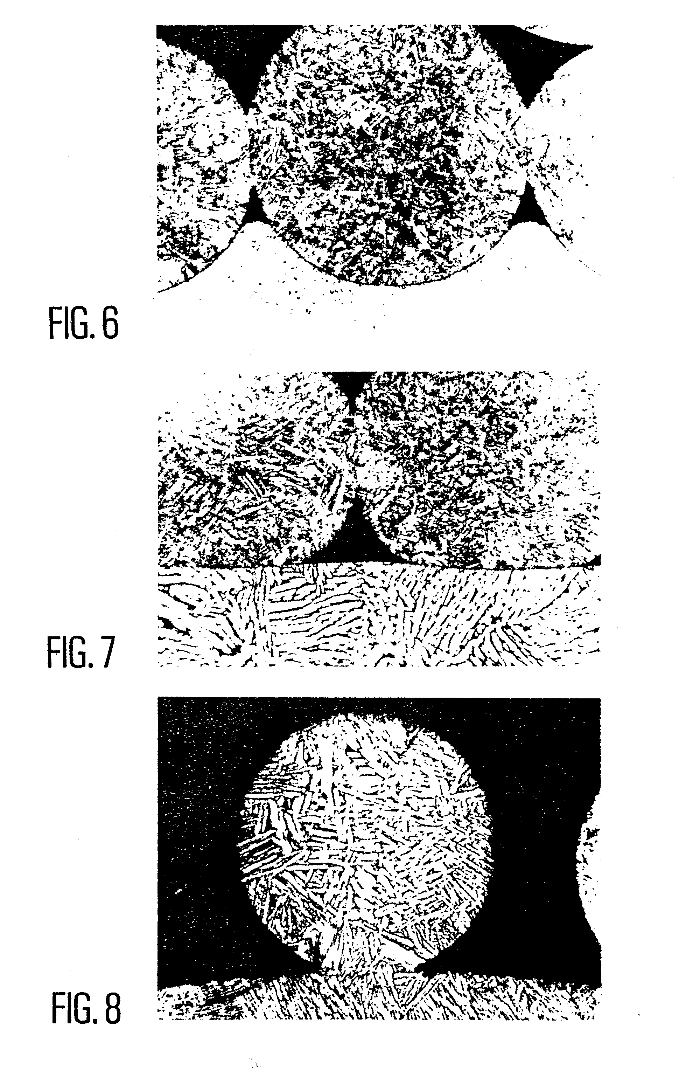

- les trois figure 6, 7 et 8 sont des coupes métallographiques illustrant les différences entre le procédé objet de l'invention et des techniques de l'art antérieur.

Comme indiqué plus haut, celui-ci consiste essentiellement à placer le substrat dans des conditions dans lesquelles son matériau constitutif est superplastique et à fritter les sphérules sur le substrat pendant qu'il se trouve dans son domaine de superplasticité.

En effet, dans certains intervalles de températures, des matériaux comme le titane ou les alliages de titane sont superplastiques, c'est-à-dire qu'ils peuvent subir des déformations de grande amplitude sous faible contrainte et ce à des températures relativement basses. Pour le titane et ses alliages, ces températures sont de l'ordre de 700 à 1000°C, ce qui permet de la déformer sous faible contrainte à des températures relativement basses. Ces déformations permettent un ancrage mécanique de la structure poreuse (qui est de préférence constituée d'un empilement de sphérules) par un phénomène d'incrustation de calottes sphériques. Cette incrustation est réalisée sans diffusion entre les sphérules et le support en disposant sur ce dernier une barrière antidiffusion constituée par exemple par une couche d'oxyde.

Le résultat obtenu apparaît sur la figure 2 où l'on voit la sphérule 10 fixée sur le substrat 12. Cependant, contrairement à ce qui se passe dans le cas de la figure 1, il n'y a pas de liaison métallurgique entre la sphérule 10 et le substrat 12. La sphérule a été en quelque sorte enfoncée dans un trou qui s'est créé à la surface du substrat grâce au fait que celui-ci est superplastique aux températures utilisées. La sphérule 10 est donc en contact avec le substrat sur une zone qui a la forme d'une calotte sphérique. Comme il n'y a pas eu de diffusion entre les sphérules et le substrat, la structure métallurgique des sphérules est différente de celle du substrat. L'absence de diffusion est due au fait qu'on a créé une barrière de diffusion à la surface du substrat, par exemple par création d'une couche d'oxyde avant de fritter la couche poreuse. On voit sur la figure 2 que les plaquettes 16 de structure $\alpha$ se trouvent uniquement à l'intérieur de la sphérule et non pas dans une zone chevauchant la sphérule et le substrat. Quant à ce dernier, il présente une structure équiaxe superplastique constituée par les grains 18.

La déformation superplastique d'un métal ou d'un alliage dépend à la fois de la dimension du grain constituant la microstructure et du facteur de forme de ces grains. Dans le cas des alliages biphasés comme les alliages de titane, elle dépend aussi des concentrations volumiques des phases en présence.

Les figures 3 et 4 illustrent les deux principales façons de disposer la couche poreuse sur le substrat 12 lorsque celui-ci est de forme cylindrique, par exemple quand il s'agit de l'âme d'une prothèse de hanche. Dans le cas de la figure 3, la couche poreuse a la forme d'un anneau cylindroconique entourant l'âme de la prothèse. La couche poreuse est frittée directement sur la surface du substrat sans usinage préalable de cette dernière. Les incrustations sont suffisantes pour éviter les ruptures par cisaillement.

Dans le cas de la figure 4, la structure poreuse n'est plus posée directement à la surface du substrat 12, mais dans une cavité ou lamage 20, ménagée à partir de la surface du substrat. Dans ce cas, on effectue toujours un frittage, mais l'ancrage de la couche poreuse est obtenu par sertissage de l'ensemble des sphérules 10 dans le logement 20. La profondeur de la cavité 20, à partir de la surface du substrat 12, est de l'ordre de 0,5 à 2mm. Quant au diamètre du substrat 12, s'il s'agit de l'âme d'une prothèse de hanche, il est de l'ordre de 10 à 20 mm.

Lorsqu'on utilise, pour réaliser le substrat 12, des alliages de titane à grains fins, l'étape (b) du procédé, c'est-à-dire le frittage de la couche poreuse pendant que le substrat se trouve dans son domaine de superplasticité, est effectuée à une température comprise entre 700 et 950°C. Le frittage est effectué dans des moules réalisés pour cette opération et reproduisant la forme du composite que l'on cherche à obtenir. Une contrainte de 10 à 30 MPa est exercée sur le moule pour obtenir d'une part la liaison des sphérules entre elles et, d'autre part, la déformation du support dans les zones où ce dernier est en contact avec la structure poreuse. Il est à noter que, s'il n'y a pas de diffusion entre les sphérules en contact avec le substrat et ce dernier grâce à la création d'une barrière antidiffusion, il y a diffusion entre une sphérule et ses voisines et c'est par diffusion qu'on obtient la liaison des sphérules entre elles.

Pour réaliser l'accrochage de la couche poreuse sur le substrat, on peut soit fritter directement les sphérules sur le substrat après avoir porté celui-ci à une température à laquelle il est superplastique, soit faire un premier frittage hors substrat à haute température (entre 1000 et 1450°C) et fixer les sphérules sur le substrat au cours d'un deuxième traitement thermique qui n'est autre que l'étape (b), c'est-à-dire un deuxième frittage alors que le substrat est dans son domaine de superplasticité.

Le procédé objet de l'invention s'applique essentiellement à la réalisation de prothèses et notamment de prothèses de hanche réhabitables. Dans ce cas, l'âme de la prothèse peut être obtenue par forgeage sous la forme d'une ébauche pouvant recevoir en surépaisseur la zone poreuse. Cette âme peut être constituée d'une barre cylindrique coudée suivant la courbure de la prothèse autour de laquelle la structure poreuse sera fixée ultérieurement.

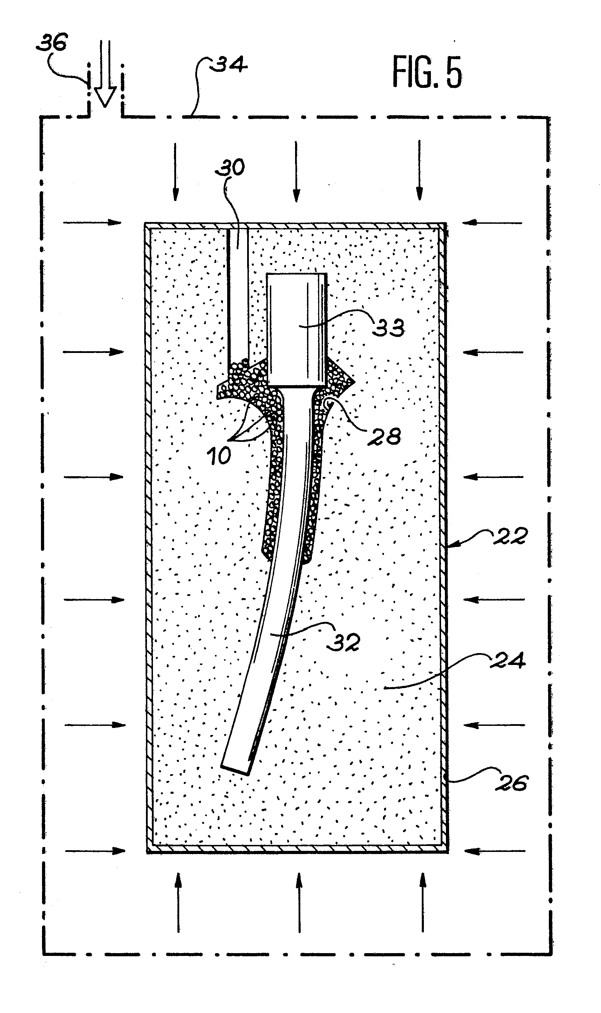

La figure 5 illustre un moule qui peut être utilisé pour la mise en oeuvre du procédé objet de l'invention. On voit que le moule 22 se compose essentiellement d'une masse en céramique 24 disposée dans une enveloppe métallique 26 pouvant être par exemple en acier mécanosoudé étanche. La masse de céramique 24 présente une cavité 28 dont la forme est celle de la prothèse à obtenir. La cavité 28 communique avec l'extérieur de l'enveloppe 26 par un canal 30. On réalise d'abord l'âme 32 de la prothèse que l'on place dans la cavité 28 du moule. Un espace existe entre au moins une partie du noyau 32 et les parois de la cavité. Les sphérules 10 sont introduites par le canal 30 jusqu'à ce qu'elles remplissent tout l'espace compris entre le noyau 32 et les parois de la cavité 28. Si nécessaire, les particules subissent un décapage avant frittage. Après dégazage sous vide, le moule 22 est porté dans un autoclave 34 représenté schématiquement en traits mixtes sur la figure 5. Une conduite 36 permet d'envoyer un gaz sous pression, par exemple de l'argon, à l'intérieur de l'autoclave 34 pour réaliser le frittage de la couche poreuse alors que le noyau 32 est dans son domaine de superplasticité.

La réalisation d'une prothèse de hanche par le procédé objet de l'invention en utilisant un dispositif tel que celui de la figure 5 peut se dérouler de la manière suivante :

On réalise d'abord le noyau 32 en coudant une barre forgée en Ti6A14V de diamètre 10 mm suivant la forme de la prothèse à obtenir. Un décolletage permet de prévoir la tête fémorale 33 qui sera usinée ultérieurement. Ce noyau a été placé dans un moule en céramique tel que celui de la figure 5 ne présentant pas à 1000°C de réactivité avec le matériau constitutif du substrat et de la couche poreuse. La porosité ouverte du moule est comprise entre 50 et 70%. On introduit ensuite dans la cavité 28 des particules de titane ou d'alliage de titane Ti6A14V. Ces particules sont sphériques et ont un diamètre compris entre 100 et 1000 micromètrès, de préférence entre 500 et 600 micromètres. En effet, c'est avec de telles dimensions qu'on peut obtenir une porosité ouverte particulièrement favorable à l'ostéogénèse. Avant frittage, les particules sont décapées dans une solution fluoronitrique pendant 20 minutes, puis rincées à l'alcool éthylique et séchées. Il est à remarquer que le noyau 32 peut être utilisé soit à l'état brut d'usinage, soit être oxydé par oxydation ménagée de sa surface. Après dégazage sous vide, le moule 22 est placé dans l'autoclave 34 sous une pression d'argon de 20 MPa pendant 4 heures pour réaliser la déformation du substrat et l'incrustation de la zone poreuse ainsi que la liaison par diffusion des particules d'alliage de titane entre elles.

La figure 6 illustre, selon l'invention, une incrustation de sphérules dans le domaine superplastique d'un support, c'est-à-dire avec des grains fins obtenus par forgeage. Il n'y a pas de liaison métallurgique entre les sphérules et le support qui est en alliage Ti6A14V forgé, avec une cristallisation α fine équiaxe. La qualité de l'incrustation montre nettement que l'état superplastique du support lors de la mise en oeuvre du procédé est nettement identifiable.

La figure 7 est relative à un support en alliage Ti6A14V fritté, cristallisé dans le système α en "vannerie". Cette figure montre clairement que l'incrustation ne peut pas être obtenue dans un support qui ne possède pas les caractéristiques superplastiques, c'est-à-dire avec des grains grossiers obtenus par frittage (les conditions de traitement thermomécaniques ayant permis la réalisation des structures des figures 6 à 7 sont identiques).

La figure 8 montre la liaison d'une particule sur un support par brasure à l'argent. Le support est en alliage Ti6A14V forgé, ayant donné lieu à une cristallisation aciculaire β transformée. La coupe métallographique fait apparaître clairement une continuité métallurgique entre la structure des sphérules et le support, quelle que soit la structure de ce dernier. Dans ce cas, le support est fragilisé par un effet d'entaille bien connu dans les alliages de titane.

Les deux dernières figures 7 et 8, qui n'appartiennent pas à l'invention permettent de comprendre immédiatement l'originalité de celle-ci.

Ainsi, le procédé objet de l'invention présente des avantages particulièrement intéressants puisqu'il permet d'incruster de manière sûre et durable des particules sphériques dans un substrat en évitant tous les risques de fragilisation qui existaient avec les méthodes de l'art antérieur. Ceci est particulièrement intéressant dans le domaine des prothèses et notamment des prothèses de hanche, puisque la couche poreuse ne risque pas de quitter le substrat sur lequel elle est accrochée.

Enfin, il est bien entendu que l'invention ne se limite pas au seul mode de réalisation qui vient d'être décrit ici, mais qu'on peut envisager des variantes sans sortir pour autant du cadre de l'invention. C'est ainsi que l'homme du métier pourra modifier le dispositif utilisé et les conditions expérimentales pourvu que le substrat soit porté dans un domaine de températures où il est superplastique. Enfin, si l'invention est essentiellement utilisée pour réaliser des prothèses, ce procédé s'applique également à tous les domaines où une couche poreuse doit être accrochée sur un substrat.

**Revendications**

1. Procédé de fixation d'une couche poreuse sur un substrat (12), le substrat (12) étant en un premier matériau et la couche poreuse en un deuxième matériau, caractérisé en ce qu'il comprend les étapes suivantes consistant à :
    (a) - amener le substrat (12) dans des conditions dans lesquelles le premier matériau est superplastique, et
    (b) - fritter la couche poreuse sur le substrat (12) afin que celle-ci s'incruste au moins partiellement sur la surface du substrat.

2. Procédé selon la revendication 1, caractérisé en ce que le premier matériau appartient au groupe constitué par le titane, le cobalt, le chrome, le tantale et leurs alliages.

3. Procédé selon la revendication 1, caractérisé en ce que le deuxième matériau appartient au groupe constitué par le titane, le cobalt, le chrome, le tantale et leurs alliages.

4. Procédé selon la revendication 1, caractérisé en ce que la couche poreuse est constituée de sphérules (10).

5. Procédé selon la revendication 4, caractérisé en ce que le diamètre des sphérules (10) est compris entre 100 et 1000 micromètres et de préférence entre 500 et 600 micromètres.

6. Procédé selon la revendication 1, caractérisé en ce que, le premier matériau étant du titane, l'étape (b) est effectuée à une température comprise entre 700 et 950°C et de préférence à 825°C.

7. Procédé selon la revendication 1, caractérisé en ce que l'étape (b) est effectuée sous une pression comprise entre 10 et 30 Mpa et de préférence égale à 20 MPa.

8. Procédé selon la revendication 1, caractérisé en ce qu'il comporte une étape supplémentaire (c), effectuée avant l'étape (b), consistant à créer une barrière antidiffusion à la surface du substrat (12).

9. Procédé selon la revendication 8, caractérisé en ce que l'étape (c) est réalisée par création d'une couche d'oxyde à la surface du substrat (12).

10. Procédé selon la revendication 1, caractérisé en ce qu'il comprend une étape supplémentaire (d), effectuée avant l'étape (b), consistant à fritter la couche poreuse hors substrat (12) à haute température.

11. Procédé selon la revendication 10, caractérisé en ce que, le deuxième matériau étant du titane, l'étape (d) est effectuée à une température comprise entre 1000 et 1450°C et de préférence à 1250°C.

12. Application du procédé selon l'une quelconque des revendications 1 à 11 à la réalisation d'une prothèse.

FIG. 1

FIG. 2

FIG. 3

FIG. 4

FIG. 5

0280592

FIG. 6

FIG. 7

FIG. 8